# EUROPÄISCHE PATENTSCHRIFT

(11) **EP 2 061 752 B1**
(45) Veröffentlichungstag und Bekanntmachung des Hinweises auf die Patenterteilung: **13.06.2012**
(21) Anmeldenummer: 07801696.1
(22) Anmeldetag: 16.08.2007
(51) Int. Cl.: C07C 253/04, C07C 253/32, C07C 255/05

(54) **VERFAHREN ZUR HERSTELLUNG VON ALKALI- ODER ERDALKALIMETALLTRICYANOMETHANIDEN**
PROCESS FOR PREPARING ALKALI METAL OR ALKALINE EARTH METAL TRICYANOMETHANIDES
PROCÉDÉ DE PRÉPARATION DE TRICYANOMÉTHANIDES DE MÉTAL ALCALIN OU ALCALINO-TERREUX

(30) Priorität: 16.08.2006 EP 06017027
(43) Veröffentlichungstag der Anmeldung: 27.05.2009
(73) Patentinhaber: Lonza AG, 4052 Basel (CH)
(72) Erfinder: TÄSCHLER, Christoph, 3912 Termen (CH); BREUER, Andreas, 3905 Saas Almagell (CH)
(86) Internationale Anmeldenummer: PCT/EP2007/007240
(87) Internationale Veröffentlichungsnummer: WO 2008/019852

(56) Entgegenhaltungen:
- WO-A-98/29389
- SCHMIDTMANN H: "UEBER EINIGE DERIVATE DES MALONITRILS" CHEMISCHE BERICHTE, Bd. 29, 1896, Seiten 1168-1175, XP009044882 ISSN: 0009-2940 in der Anmeldung erwähnt
- COX EDWARD, FONTAINE ANDRE: "Le cyanoforme ou tricyanométhane. -Nouvelle méthode de préparation" BUL.SOC.CHIM.FR., 1954, Seiten 948-950, XP009085459 in der Anmeldung erwähnt

## Beschreibung

Die vorliegende Erfindung betrifft ein Verfahren zur Herstellung von Alkali- oder Erdalkalimetalltricyanomethaniden mit einer besonders hohen Reinheit, insbesondere einer hohen Halogenidfreiheit.

Ionische Flüssigkeiten, enthaltend Alkali- oder Erdalkalimetalltricyanomethanide (TCM), sind für die elektrischen Industrie wichtige Roh- und Hilfsstoffe, u.a. für die Herstellung von wiederaufladbaren Batterien und elektronischen Bausteinen. Für diese Anwendungsgebiete ist es erforderlich, dass die verwendeten Methanide besonders rein, insbesondere halogenidfrei, sind, um Korrosionsprobleme und/oder ungewollte Nebenreaktionen zu vermeiden.

Die Herstellung von Tricyanomethaniden ist bekannt. Die Cyanidierung von Malonodinitril (MDN) wurde erstmal von Schmidtmann in Ber. Dtsch. Chem. Ges. 1896, 29, 1168 beschrieben. Hierbei wird MDN mit Natriumethanolat in Ethanol deprotoniert und durch schrittweise Zugabe von Chlorcyan zu Natriumtricyanomethanid umgesetzt und dann aus Ether kristallisiert. Bei dem Verfahren wird Natriumtricyanomethanid in einer Ausbeute von ca. 70% isoliert.

Birckenbach et al. offenbarten in Chem. Ber. 1929, 62B, 153 die Cyanidierung von MDN mit Bromcyan. Sowohl Birckenbach et al. als auch Mayer et al. (Monatsh. Chem., 1969, 100, 462) haben die Herstellung von halogenarmem Silbertricyanomethanid beschrieben, indem rohes Alkalimetalltricyanomethanid mit Silbernitrat versetzt wurde, wobei zuerst Silberchlorid oder Silberbromid ausfällt. Aus dem Filtrat konnte nach weiterer Silbernitratzugabe Silbertricyanomethanid isoliert werden. Mayer et al. haben im Weiteren die Umsetzung von Silbertricyanomethanid mit Chlorcyan über 40 Stunden bei 100 °C zu Tetracyanomethan beschrieben, welches dann sublimiert und anschliessend in Schwefelsäure zu Ammoniumtricyanomethanid hydrolysiert wurde. Lithiumtricyanomethanid wurde von Mayer et al. durch Zugabe von Lithiumchlorid zu einer Acetonitrillösung von Tetracyanomethan bei -96 °C erhalten.

Die Herstellung hochreinen Kaliumtricyanomethanids wurde erstmals von Hipps et al. offenbart (J. Phys. Chem. 1985, 89, 5459). In dem Verfahren wurde Kaliumtricyanomethanid 10-mal umkristallisiert. Um Reste von organischen Verunreinigungen zu entfernen wurde das erhaltene Kaliumtricyanomethanid anschliessend noch zweimal aus Wasser umkristallisiert. Hierbei wurde ein weisses kristallines Pulver erhalten, das bei einer Anregung mit Licht von bei 5145Å keinen Raman-Fluoreszenz Hintergrund zeigte und als hochrein interpretiert wurde.

In WO-A-98/29389 wurde die Cyanidierung von MDN mit Bromcyan in Gegenwart von 1,4-Diazabicyclo[2.2.2]octan (DABCO^{®}) in THF offenbart. Bei dem Verfahren wird DABCO^{®}-Hydrochlorid innerhalb von 28 h bei -20°C auskristallisiert. Hierbei wurde 98%-iges Lithiumtricyanomethanid erhalten.

Ein weiteres Verfahren wurde von Trofimenko et al. in J. Org. Chem. 1962, 27, 433 offenbart, worin Kaliumtricyanomethanid durch Behandlung eines Dihalogenmalonodinitrilkaliumbromid-Komplexes mit Kaliumcyanid erhalten wurde. Das Verfahren liefert Ausbeuten bis 60%.

Fox et al. haben in Bull. Soc. Chim. Fr. 1954, 948 ein weiteres Verfahren zur Herstellung von Tricyanomethan bei tiefer Temperatur beschrieben, wobei Brommalonodinitril mit Kaliumcyanid umgesetzt wurde.

Weitere Verfahren zur Herstellung von Tricyanomethaniden, enthaltend die Umsetzung von deprotoniertem MDN mit Phenylcyanat wurden von Grigat et al. in Chem. Ber. 1965, 98, 3777-3784 und Martin et al. in DD-A-48614 offenbart. Hierbei wurden Ausbeuten von 75 bis 88% erhalten.

Die bekannten Verfahren liefern jedoch Produkte, die nicht halogenfrei sind und immer in mehr oder weniger aufwändigen Reinigungsschritten nachbearbeitet werden müssen.

Ein Verfahren zur Reinigung von Natriumtricyanomethanid durch Umkristallisation in Acetonitril wurde 1987 von Bock et al. in Z. Naturforsch., 1987, 42b, 315 beschrieben.

Hierbei wurde Natriumtricyanomethanid in 70%-iger Ausbeute erhalten. Die erreichte Reinheit wird jedoch nicht durch genaue Angaben belegt.

Aufgabe der vorliegenden Erfindung war die Bereitstellung eines einfachen und schnellen Verfahrens zur Herstellung von hochreinen Tricyanomethaniden. Das Verfahren sollte sich für die grosstechnische Produktion eignen.

Die Aufgabe wurde entsprechend Anspruch 1 gelöst.

Beansprucht wird ein Verfahren zur Herstellung von Alkali- oder Erdalkalitricyanomethaniden in einer Reinheit von mindestens 99 Gew.-% und einem Halogenidgehalt von höchstens 20 ppm, worin in einem ersten Schritt Malonodinitril in Gegenwart von Wasser und einer Säure durch parallele Zugabe von einem Halogencyan oder Dicyan, sowie einer Alkalimetallbase zum entsprechenden Alkali- oder Erdalkalimetalltricyanomethanid umgesetzt wird und mindestens während der Zugabe des Halogen- oder Dicyans ein pH-Wert von 4,0 bis 9,5, vorzugsweise von 6,4 bis 7,7, ganz besonders bevorzugt von 7,0 bis 7,6 aufrecht erhalten wird, und worin das zunächst gebildete Alkali- oder Erdalkalimetalltricyanomethanid in einem zweiten Schritt in einem anderen Lösungsmittel umkristallisiert wird.

Überraschenderweise wurde gefunden, dass die Durchführung der Cyanidierung bei Alkali- oder Erdalkalimetalltricyanomethanidkonzentrationen von 22 bis 24 Gew.-% das Alkalimetalltricyanomethanid grösstenteils in Form feiner Kristalle ausfällt und direkt mit ca. 60%-iger Ausbeute als Feststoff isoliert werden kann.

Wesentliche Merkmale der Erfindung sind der Wechsel der Lösungsmittel zwischen Synthese und Aufreinigung, sowie die Bedingungen in der Reaktionsführung. Die Cyanidierung wird in wässriger Lösung in einem engen pH-Bereich durchgeführt, wobei nur wenige Nebenprodukte gebildet werden, welche sich dann sehr effizient mit einer Umkristallisation in einem geeigneten organischen Lösungsmittel entfernen lassen.

Vorzugsweise wird das Malononitril in Wasser in Gegenwart eines Puffersystems gelöst, wobei die Herstellung des Puffersystems vorzugsweise durch Zugabe einer Säure und nachfolgender Einstellung des pH-Wertes mit einer Base erfolgt. Die Durchführung der Reaktion in Gegenwart eines Puffersystems erleichtert wesentlich die Aufrechterhaltung eines engen pH-Bereichs. In einer bevorzugten Ausführungsform wird die Säure ausgewählt aus der Gruppe bestehend aus mit Wasser mischbaren organischen und anorganischen Säuren, von Wasser benetzbaren organischen und anorganischen Polysäuren und Mischungen daraus. Geeignete Säuren werden ausgewählt aus der Gruppe bestehend aus Ameisensäure, Essigsäure, Chloressigsäure, Propionsäure, 2-Chlorpropionsäure, 3-Chlorpropionsäure, Toluolsulfonsäure, Phosphorsäure, Schwefelsäure, Salzsäure, und Salpetersäure, sauren Ionentauschern, Polyphosphorsäure und Heteropolysäuren wie Polymolybdänsäure und Polywolframsäuren. Besonders bevorzugt werden mehrbasige Säuren, ganz besonders bevorzugt Phosphorsäure verwendet. Als Base im Puffersystem wird vorzugsweise die gleiche Base verwendet, die später bei der Reaktion zugegeben wird.

Als Halogencyan kann Chlor- und Bromcyan verwendet werden. Dicyan weist durch die Anwesenheit einer Pseudohalogengruppe vergleichbare Eigenschaften zu Chlor- und Bromcyan auf. Besonders bevorzugt wird das Halogencyan oder Dicyan im Verhältnis zu Malononitril von 1:1 1 bis 10:1, vorzugsweise von 1:1: bis 1:3 verwendet. Ein Überschuss an Halogencyan oder Dicyan ist besonders bevorzugt. Vorzugsweise wird Chlorcyan verwendet.

Die Base im erfindungsgemässen Verfahren enthält mindestens ein Alkali- oder Erdlkalimetall ausgewählt aus der Gruppe bestehend aus Lithium, Natrium, Kalium, Magnesium, Calcium oder Barium. Vorzugsweise werden Lithium und/oder Natrium verwendet.

Weiterhin ist die Alkali- oder Erdalkalimetallbase besonders bevorzugt eine starke Base. Insbesondere können hierbei Alkali- oder Erdalkalimetallhydroxide, Alkali- oder Erdalkalimetalloxide oder Alkali- oder Erdalkalimetallalkoxide verwendet werden. Als Alkali- oder Erdalkalimetallalkoxide können insbesondere C₁₋₆-Alkoxide verwendet werden. In einer ganz besonders bevorzugten Ausführungsform ist die Base ein Alkali- oder Erdalkalimetallhydroxid.

Das zunächst gebildete Alkalimetalltricyanomethanid weist im erfindungsgemässen Verfahren nach dem ersten Schritt einen Halogenidgehalt bis zu 10 Gew.-% auf. Dieser Feststoff kann dann in einem Schritt in einem anderen Lösungsmittel, vorzugsweise in einem organischen Lösungsmittel umkristallisiert werden. Das Produkt kann aus der Mischung, vorzugsweise nach Abkühlung, als Feststoff erhalten werden. Alkali- oder Erdalkalimetalltricyanomethanide werden hierbei mit niedrigen Halogenidgehalten, einer hohen chemischen Reinheit und in guten Ausbeuten von 94 Gew.-% bezogen auf das eingesetzte rohe Alkali- oder Erdalkalimetalltricyanomethanid erhalten.

In einer bevorzugten Verfahrensvariante wird im zweiten Schritt das Alkali- oder Erdalkalimetalltricyanomethanid zunächst, gegebenenfalls bei erhöhter Temperatur, in Methylethylketon und/oder Methylisobutylketon gelöst und, gegebenenfalls bei erniedrigter Temperatur, ausgefällt.

In einer weiteren bevorzugten Verfahrensvariante wird das Alkali- oder Erdalkalimetalltricyanomethanid, gegebenenfalls nach vorheriger Zugabe und Abtrennung von Aktivkohle, in Gegenwart von Methyl-*tert*-butylether oder Diisopropylether, ausgefällt.

Besonders bevorzugt wird die Umkristallisation mit den Lösungsmitteln Aceton und Methyl-*tert*-butylether (MTBE) durchgeführt.

Die Umkristallisation wird in einer bevorzugten Verfahrensvariante in Gegenwart eines porösen Filterhilfsmittels wie beispielsweise Aktivkohle, Cellulose, Baumwolle, Kieselgur oder Kieselsol durchgeführt. Vorteilhaft wird das Alkali- oder Erdalkalimetalltricyanomethanid in Gegenwart des Filterhilfsmittels gelöst. Das Filterhilfsmittel kann aber auch während des Lösens oder nach Erhalt der Lösung zugegeben werden. Das Filtermaterial wird abgetrennt, bevor das Alkali- oder Erdalkalimetalltricyanomethanid ausgefällt wird. Besonders bevorzugt als Filterhilfsmittel wird Aktivkohle verwendet.

Vorzugsweise wird das Alkali- oder Erdalkalimetalltricyanomethanid bei einer Temperatur unterhalb 20 °C, besonders bevorzugt bei 10 °C oder weniger ausgefällt.

In dem erfindungsgemässen Verfahren wird nach dem zweiten Schritt ein Alkali- oder Erdalkalimetalltricyanomethanid in einer Reinheit von mindestens 99 Gew.-%, vorzugsweise von mindestens 99,5 Gew.-%, besonders bevorzugt von mindestens 99,8 Gew.-% erhalten. Ganz besonders bevorzugt können Alkali- oder Erdalkalimetalltricyanomethanide mit einer Reinheit von 99,9 Gew.-% und mehr erhalten werden. Die Reinheit in Gew.-% wird als Reinheit der lösungsmittelfreien Alkali- oder Erdalkalimetalltricyanomethanide verstanden.

In dem erfindungsgemässen Verfahren wird nach dem zweiten Schritt ein Alkali- oder Erdalkalimetalltricyanomethanid mit einem Halogenidgehalt von höchstens 20 ppm erhalten, vorzugsweise von höchstens 10 ppm, besonders bevorzugt von höchstens 5 ppm.

### Beispiele

Bei den folgenden Beispielen kann durch Ersatz der Natronlauge mit einer anderen Alkali- oder Erdalkalimetallbase das entsprechende Alkali- oder Erdalkalimetalltricyanomethanid gewonnen werden. Durch Verwendung von Gemischen von Basen unterschiedlicher Alkali- oder Erdalkalimetalle können auch gemischte Tricyanomethanide erhalten werden.

### Beispiel 1:

In einem Rührwerk werden Malonodinitril (462 g, 5,95 mol, 85%-ig in Methanol), Wasser (2021 g) und Phosphorsäure (57,1 g, 0,5 mol, 85%-ig) gemischt. Anschliessend wird mit Natronlauge (50%-ig) ein pH-Wert von 7,5 eingestellt. Bei 25 bis 30 °C wird innerhalb von 2 h Chlorcyan (979 g, 15,92 mol) zudosiert, wobei der pH-Wert durch geregelte Zugabe von Natronlauge (50%-ig) auf 6,4 bis 7,5 gehalten wird. Es bildet sich eine beige bis braune klare Lösung. Nach Zugabe der gesamten Menge an Chlorcyan wird die Reaktionsmischung noch für weitere 30 min bei 25 bis 30 °C gerührt. Hierbei wird der pH-Wert durch geregelte Zugabe von Natronlauge (50%-ig) auf 7,0 bis 7,5 gehalten. Anschliessend wird mit Natronlauge ein pH von 8,5 eingestellt. Dann werden 50 g Aktivkohle zur Reaktionsmischung gegeben. Die erhaltene Suspension wird für weitere 30 min bei 25 bis 30 °C gerührt und anschliessend filtriert. Als Filtrat erhält man 3950 g einer gelblichen Lösung aus bestehend aus 16,8 Gew.-% Natriumtricyanomethanid, 10,5 Gew.-% anorganischen Salzen, 71,5 Gew.-% Wasser, 1,3 Methanol und 0,1 Gew.-% organischer Verunreinigungen. Die Reinheit des Produktes beträgt 98,6 Gew.-%.

### Beispiel 2:

In einem Rührwerk werden Malonodinitril (513 g, 6,61 mol, 85%-ig in Methanol), Wasser (2021 g) und Phosphorsäure (63,5 g, 0,55 mol, 85%-ig,) gemischt. Mit Natronlauge (50%-ig) wird ein pH-Wert von 7,2 eingestellt. Bei 25 bis 30 °C wird innerhalb von 3 h Chlorcyan (1098 g, 17,85 mol) zudosiert, wobei der pH-Wert durch geregelte Zugabe von Natronlauge (50%-ig) auf 7,0 bis 7,2 gehalten wird. Es bildet sich eine beige bis braune klare Lösung. Nachdem die gesamte Menge an Chlorcyan zugegeben worden ist, wird die Reaktionsmischung noch für weitere 30 min bei 25 bis 30 °C gerührt. Hierbei wird der pH-Wert durch geregelte Zugabe von Natronlauge (50%-ig) auf 7,0 bis 7,2 gehalten. Danach wird mit Natronlauge ein pH von 8,5 eingestellt und es werden 50 g Aktivkohle zur Reaktionsmischung gegeben. Die erhaltene Suspension wird bei 25 bis 30 °C für weitere 30 min gerührt und anschliessend abfiltriert. Es werden 4,17 kg einer gelblichen Lösung aus Natriumtricyanomethanid in Wasser mit einem Gehalt von 17,9 Gew.-% Natriumtricyanomethanid erhalten. Ausserdem sind noch 11,0 Gew.-% anorganische Salze, 1,0 Gew.-% Methanol, 69,9 Gew.-% Wasser und 0,2 Gew.-% organischer Verunreinigungen enthalten. Die Ausbeute von Natriumtricyanomethanid bezogen auf das eingesetzte Malonodinitril beträgt 100%. Die Reinheit des Produktes beträgt über 98,8 Gew.-%.

### Beispiel 3:

Malonodinitril (169,6 g, 2,18 mol, 85%-ig in Methanol), Wasser (392,8 g) und Phosphorsäure (18,8 g, 0,16 mol 85%-ig) werden gemischt. Anschliessend wird mit Natronlauge (50%-ig) ein pH-Wert von 7,5 eingestellt. Bei 25 bis 30°C wird innerhalb von 4 h Chlorcyan (137,8 g, 2,24 mol) zudosiert, wobei der pH-Wert durch geregelte Zugabe von Natronlauge (50%-ig) auf 7,3 bis 7,5 gehalten wird. Nachdem die gesamte Menge an Chlorcyan zugegeben ist, wird die beige bis braune Reaktionsmischung noch für weitere 30 min bei 25 bis 30°C gerührt, wobei der pH Wert durch geregelte Zugabe von Natronlauge (50%-ig) auf 7,3 bis 7,5 gehalten wird. Danach wird der pH Wert mit Natronlauge auf 8,5 eingestellt und die Temperatur auf 70 °C erhöht. Dabei bildet sich aus der Suspension wieder eine klare beige bis braune Lösung. Diese Lösung wird dann mit einer Rate von 6 °C/h auf 10 °C abgekühlt wobei sich wieder eine Suspension bildet, die anschliessend zentrifugiert wird. Auf diese Weise werden 167,9 g feuchtes Natriumtricyanomethanid mit einem Natriumtricyanomethanidgehalt von 72 Gew.-% erhalten. Dies entspricht einer Ausbeute von 49%. Aus der Mutterlauge können weitere 47 Gew.-% Natriumtricyanomethanid erhalten werden. Weiterhin sind in dem Feuchtprodukt 2,6 Gew.-% Natriumchlorid, 24,0 Gew.-% Wasser und 1,2 Gew.-% organische Verunreinigungen enthalten. Die Reinheit des Produktes beträgt ca. 98,8 Gew.-%.

### Beispiel 4:

Feuchtes Natriumtricyanomethanid gemäss Beispiel 3 (717 g, 4,08 mol, 64,3%-ig, enthaltend ca. 2,7 Gew.-% Natriumchlorid) wird bei 15 mbar und 55 °C für 12 Stunden getrocknet, wobei 519 g getrocknetes Rohprodukt erhalten wird. Dieses Rohprodukt wird in Aceton (3 L) bei 45 °C gelöst, wobei eine braune Lösung erhalten wird, die dann mit Aktivkohle (112 g) versetzt wird. Nach 15 min Rühren bei 25 °C und anschliessender Filtration erhält man eine klare leicht gelbliche Lösung. Diese Lösung wird mit Methyl-tert-butylether (MTBE, 9 L) versetzt und auf 10 °C abgekühlt, wobei eine weisse Suspension entsteht. Der abgenutschte Rückstand wird bei 15 mbar und 40 °C für 8 Stunden getrocknet. Es wird weisses Natriumtricyanomethanid (432 g, 3,82 mol, 94% Ausbeute) mit einem Gehalt von 99,9 Gew.-% erhalten. Der Chloridgehalt dieses Produktes beträgt weniger als 5 ppm.

## Patentansprüche

1. Ein Verfahren zur Herstellung von Alkali- oder Erdalkalimetalltricyanomethaniden in einer Reinheit von mindestens 99 Gew.-% und einem Halogenidgehalt von höchstens 20 ppm, worin in einem ersten Schritt Malonodinitril in Gegenwart von Wasser und einer Säure durch parallele Zugabe von Halogencyan oder Dicyan, sowie einer Alkali- oder Erdalkalimetallbase zum entsprechenden Alkali- oder Erdalkalimetalltricyanomethanid umgesetzt wird und mindestens während der Zugabe des Halogencyans ein pH-Wert von 4,0 bis 9,5 aufrecht erhalten wird, und worin das zunächst gebildete Alkali- oder Erdalkalimetalltricyanomethanid in einem zweiten Schritt in einem anderen Lösungsmittel umkristallisiert erhalten wird.

2. Verfahren nach Anspruch 1, worin das Malonodinitril zuerst in Wasser gelöst und danach mit einer Säure gemischt wird.

3. Verfahren nach Anspruch 1 oder 2, **dadurch gekennzeichnet, dass** die Säure eine anorganische Säure ist.

4. Verfahren nach einem der Ansprüche 1 bis 3, **dadurch gekennzeichnet, dass** das Halogencyan Chlorcyan ist.

5. Verfahren nach einem der Ansprüche 1 bis 4, **dadurch gekennzeichnet, dass** die Alkali- oder Erdalkalimetallbase mindestens ein Alkali- oder Erdalkalimetall ausgewählt aus der Gruppe bestehend aus Lithium, Natrium, Kalium, Calcium, Magnesium und Barium enthält.

6. Verfahren nach einem der Ansprüche 1 bis 5, **dadurch gekennzeichnet, dass** die Alkali- oder Erdalkalimetallbase ausgewählt ist aus der Gruppe bestehend aus Alkali- oder Erdalkalimetallhydroxiden, Alkali- oder Erdalkalimetalloxiden und Alkali- oder Erdalkalimetallalkoxiden.

7. Verfahren nach einem der Ansprüche 1 bis 6, **dadurch gekennzeichnet, dass** im zweiten Schritt Alkali- oder Erdalkalimetalltricyanomethanid zunächst, gegebenenfalls bei erhöhter Temperatur, in Aceton, Methylethylketon und/oder Methylisobutylketon gelöst und anschliessend durch Zugabe von Methyl-tert-butylether oder Diisopropylether, gegebenenfalls bei erniedrigter Temperatur, ausgefällt wird.

8. Verfahren nach Anspruch 7, **dadurch gekennzeichnet, dass** im zweiten Schritt der Alkali- oder Erdalkalimetalltricyanomethanidlösung ein poröses Filtermaterial zugesetzt wird, der vor dem Ausfällen der Alkali- oder Erdalkalimetalltricyanomethanide abfiltriert wird.

9. Verfahren nach Anspruch 7 oder 8, worin das Alkali- oder Erdalkalimetalltricyanomethanid durch Zugabe von Methyl-tert-butylether oder Diisopropylether ausgefällt wird.

10. Verfahren nach einem der Ansprüche 1 bis 9, worin Alkali- oder Erdalkalimetalltricyanomethanid in einer Reinheit von mindestens 99,5 Gew.-% erhalten wird, besonders bevorzugt in einer Reinheit von mindestens 99,8 Gew.-%.

## Claims

1. A process for preparing alkali metal or alkaline earth metal tricyanomethanides having a purity of at least 99% by weight and a halide content of not more than 20 ppm, wherein, in a first step, malononitrile is converted into the corresponding alkali metal or alkaline earth metal tricyanomethanide in the presence of water and an acid by parallel addition of a halocyan or dicyan and also an alkali metal or alkaline earth metal base, and wherein a pH of from 4.0 to 9.5 is maintained at least during the addition of the halocyan, and the alkali metal or alkaline earth metal tricyanomethanide initially formed is recrystallized from a different solvent in a second step.

2. The process as claimed in claim 1, wherein the malononitrile is firstly dissolved in water and then mixed with an acid.

3. The process as claimed in claim 1 or 2, **characterized in that** the acid is an inorganic acid.

4. The process as claimed in any of claims 1 to 3, **characterized in that** the halocyan is chlorocyan.

5. The process as claimed in any of claims 1 to 4, **characterized in that** the alkali metal or alkaline earth metal base contains at least one alkali metal or alkaline earth metal selected from the group consisting of lithium, sodium, potassium, calcium, magnesium and barium.

6. The process as claimed in any of claims 1 to 5, **characterized in that** the alkali metal or alkaline earth metal base is selected from the group consisting of alkali metal and alkaline earth metal hydroxides, alkali metal and alkaline earth metal oxides and alkali metal and alkaline earth metal alkoxides.

7. The process as claimed in any of claims 1 to 6, **characterized in that**, in the second step, alkali metal or alkaline earth metal tricyanomethanide is firstly dissolved, if appropriate at elevated temperature, in acetone, methyl ethyl ketone and/or methyl isobutyl ketone and subsequently precipitated by addition of methyl tert-butyl ether or diisopropyl ether, if appropriate at a reduced temperature.

8. The process as claimed in claim 7, **characterized in that**, in the second step, a porous filter material is added to the alkali metal or alkaline earth metal tricyanomethanide solution and is filtered off before precipitation of the alkali metal or alkaline earth metal tricyanomethanides.

9. The process as claimed in claim 7 or 8, wherein the alkali metal or alkaline earth metal tricyanomethanide is precipitated by addition of methyl tert-butyl ether or diisopropyl ether.

10. The process as claimed in any of claims 1 to 9, wherein alkali metal or alkaline earth metal tricyanomethanide is obtained in a purity of at least 99.5% by weight, particularly preferably in a purity of at least 99.8% by weight.

## Revendications

1. Procédé de fabrication de tricyanométhanides de métaux alcalins ou alcalino-terreux ayant une pureté d'au moins 99 % en poids et une teneur en halogénures d'au plus 20 ppm, dans lequel, lors d'une première étape, du malonodinitrile est transformé en présence d'eau et d'un acide par ajout parallèle d'halogénocyan ou de dicyan, ainsi que d'une base de métal alcalin ou alcalino-terreux, en le tricyanométhanide de métal alcalin ou alcalino-terreux correspondant et, au moins pendant l'ajout de l'halogénocyan, un pH de 4,0 à 9,5 est maintenu, et dans lequel le tricyanométhanide de métal alcalin ou alcalino-terreux tout d'abord formé est recristallisé dans un autre solvant lors d'une seconde étape.

2. Procédé selon la revendication 1, dans lequel le malonodinitrile est tout d'abord dissous dans de l'eau, puis mélangé avec un acide.

3. Procédé selon la revendication 1 ou 2, **caractérisé en ce que** l'acide est un acide inorganique.

4. Procédé selon l'une quelconque des revendications 1 à 3, **caractérisé en ce que** l'halogénocyan est le chlorocyan.

5. Procédé selon l'une quelconque des revendications 1 à 4, **caractérisé en ce que** la base de métal alcalin ou alcalino-terreux contient au moins un métal alcalin ou alcalino-terreux choisi dans le groupe constitué par le lithium, le sodium, le potassium, le calcium, le magnésium et le baryum.

6. Procédé selon l'une quelconque des revendications 1 à 5, **caractérisé en ce que** la base de métal alcalin ou alcalino-terreux est choisie dans le groupe constitué par les hydroxydes de métaux alcalins ou alcalino-terreux, les oxydes de métaux alcalins ou alcalino-terreux et les alcoxydes de métaux alcalins ou alcalino-terreux.

7. Procédé selon l'une quelconque des revendications 1 à 6, **caractérisé en ce que**, lors de la seconde étape, le tricyanométhanide de métal alcalin ou alcalino-terreux est tout d'abord dissous, éventuellement à température élevée, dans de l'acétone, de la méthyléthylcétone et/ou de la méthylisobutylcétone, puis précipité par ajout d'éther de méthyle et de tert-butyle ou d'éther de diisopropyle, éventuellement à température réduite.

8. Procédé selon la revendication 7, **caractérisé en ce que**, lors de la seconde étape, un matériau de filtration poreux est ajouté à la solution de tricyanométhanide de métal alcalin ou alcalino-terreux, qui est éliminé par filtration avant la précipitation des tricyanométhanides de métaux alcalins ou alcalino-terreux.

9. Procédé selon la revendication 7 ou 8, dans lequel le tricyanométhanide de métal alcalin ou alcalino-terreux est précipité par ajout d'éther de méthyle et de tert-butyle ou d'éther de diisopropyle.

10. Procédé selon l'une quelconque des revendications 1 à 9, dans lequel le tricyanométhanide de métal alcalin ou alcalino-terreux est obtenu en une pureté d'au moins 99,5 % en poids, de manière particulièrement préférée en une pureté d'au moins 99,8 % en poids.
